Europäisches Patentamt

European Patent Office

Office européen des brevets

(11) Publication number: **0 329 854 B1**

(12) **EUROPEAN PATENT SPECIFICATION**

(45) Date of publication of patent specification: **19.11.92**  (51) Int. Cl.⁵: **A61F 2/40**

(21) Application number: **88121663.4**

(22) Date of filing: **24.12.88**

(54) Modular shoulder prothesis.

(30) Priority: **02.02.88 US 151335**

(43) Date of publication of application:
**30.08.89 Bulletin 89/35**

(45) Publication of the grant of the patent:
**19.11.92 Bulletin 92/47**

(84) Designated Contracting States:
**AT BE CH DE ES FR GB GR IT LI LU NL SE**

(56) References cited:
EP-A- 0 278 807    DE-A- 1 930 353
DE-A- 2 933 961    FR-A- 2 579 454
US-A- 3 916 451    US-A- 4 261 062

(73) Proprietor: **Dines, David M.**
**33 Foxhollow Lane**
**Old Westbury New York 11568(US)**

Proprietor: **Warren, Russell F.**
**121 Bedford Road**
**Greenwich Connecticut 06831(US)**

(72) Inventor: **Dines, David M.**
**33 Foxhollow Lane**
**Old Westbury New York 11568(US)**
Inventor: **Warren, Russell F.**
**121 Bedford Road**
**Greenwich Connecticut 06831(US)**

(74) Representative: **Cohausz & Florack Patentan-**
**wälte**
**Postfach 14 01 61 Schumannstrasse 97**
**W-4000 Düsseldorf 1(DE)**

Rank Xerox (UK) Business Services

# Description

BACKGROUND OF THE INVENTION

The present invention relates to a shoulder prosthesis for a total shoulder replacement.

Prosthesis for use in a total shoulder replacement are known and essentially consists of a humeral component which is implanted in the proximal humerus and the glenoid component which replaces the socket of the shoulder.

A shoulder prosthesis is known from FR-A-2 579 454 offering a humeral compound and a glenoid component wherein the humeral component comprising an elongated stem, a semispherical head and means removably connecting the head to one end of the stem. The connecting means comprise screw bolt, and aperture on the head and a neck on the stem.

The known shoulder prosthesis has a lot of disadvantages. Because the smaller volume in a shoulder joint, the thickness of material in such a prosthesis must be remarkable smaller, than in a prosthesis of a hip joint. Therefore the elements of this known prosthesis, especially the semispherical head and the means connecting the head to one end of the stem must be very small. This causes the danger of breakage, especially in the range of the screw bolt. Moreover the sharp edges of the semispherical head, especially at the cavitation, provided to drill in the screw, will cause pain, because they will press against, or cut in the tissue of the shoulder, when the arm is moved.

Also the edges of the bore for the screw may cause pain.

Furthermore, the connection cannot be removable by a surgeon in order to replace the head later after being implanted. It will also be very difficult, to remove worn out parts of the prosthesis, especially it may be very difficult, to find, to clean and to drill out the screw grown into tissue.

While modularity has been utilized in shoulder prosthesis with respect to the glenoid component as can be seen in the McNab-English Total Replacement developed by the De Puy Incorporated, the question of modularity has not been addressed with regard to the humeral component.

The main object of the present invention is to provide a modular shoulder prosthesis wherein the humeral component has a modular design which enables different available sized heads to be placed onto a stem which has been implanted in the proximal humerus.

"A shoulder prosthesis, comprising a glenoid component (30) and a humeral component (10, 20), the humeral component having an elongated stem (10), a semispherical head (20) and means removably connecting the head (20) to the proximal end of the stem (10), the distal portion of the stem (10) being for insertion into a humerus, characterized in that the proximal end portion of the elongated stem has a plane (18) having a raised collar with a planar upper surface (15) formed thereon, said planar upper surface (15) having a tapered aperture (16) formed therein and extending downwardly through said collar and into said proximal end portion of the stem; and

that the semispherical head (20) has a concave recessed inner surface, said inner surface having a tapered pin (22) extending from a bottom portion of said recess and outwardly thereof and dimensioned so as to form a clearance therearound with said inner surface, wherein the recess is configured to receive and surround the raised collar when said tapered pin (22) is tightly received in said aperture (16) thereby securing said joint head (20) to the stem (10)."

This modular component mechanism allows the surgeon to better set the tensions of the soft tissues about the shoulder during the procedure. This is a definite advantage in total shoulder replacement.

In addition, in those cases of fractures of the proximal humerus in which implant is used, the modular aspect of the head and stem design affords the surgeon the ability to make revisions to the size of the head if required in the future, without the need for removing the stem implant from the humerus, since at that time the humeral head can simply be removed and the socket or glenoid portion of the shoulder can then be adapted accordingly.

According to an improvement of the shoulder prosthesis according to the present invention the stem staples in cross section from the proximal end toward the distal end. Furthermore, the distal end portion of the stem should be rounded. It is furthermore advantageous that the stem is covered with a porous coating.

According to another improved embodiment of the shoulder prosthesis according to the invention the stem shall have a flange in the vicinity of said proximal end portion thereof with fixation holes therein.

According to a special embodiment the stem should have a length in the range of 70 to 115 mm and a width of from 6 to 11 mm. The head should have a diameter from 20 to 48 mm and a thickness of 15 to 27 mm whereas the stem defines a longitudinal axis and the planar surface extends from 45° to 60° to the axis of the stem.

This removable connection is preferably carried out by a coupling mechanism for the head and stem which comprises a Morse taper so that the humeral head can be inserted into the stem via a neck portion which fits into the stem.

In addition to the modular humeral component, the glenoid component can also be made modular as has been disclosed in the prior art with the result that the entire shoulder replacement is modular.

These and other features and advantages of the present invention will be described in connection with the preferred embodiments of the present invention with reference to the attached drawings, wherein:

## BRIEF DESCRIPTION OF THE DRAWINGS

Fig. 1      is a side view of the stem and head portions of a humeral component according to the present invention with a reverse Morse taper;

Fig. 2      is a front view of the stem portion of the humeral component according to the present invention;

Fig. 3      is a sectional view along line 3-3 in Fig. 1;

Fig. 4      is a top view of the stem portion of Fig. 1.

Fig. 5      is a top view of the glenoid component; and

Fig. 6      is a sectional view of the glenoid component of Fig. 5 with a plastic insert.

## DETAILED DESCRIPTION OF THE INVENTION

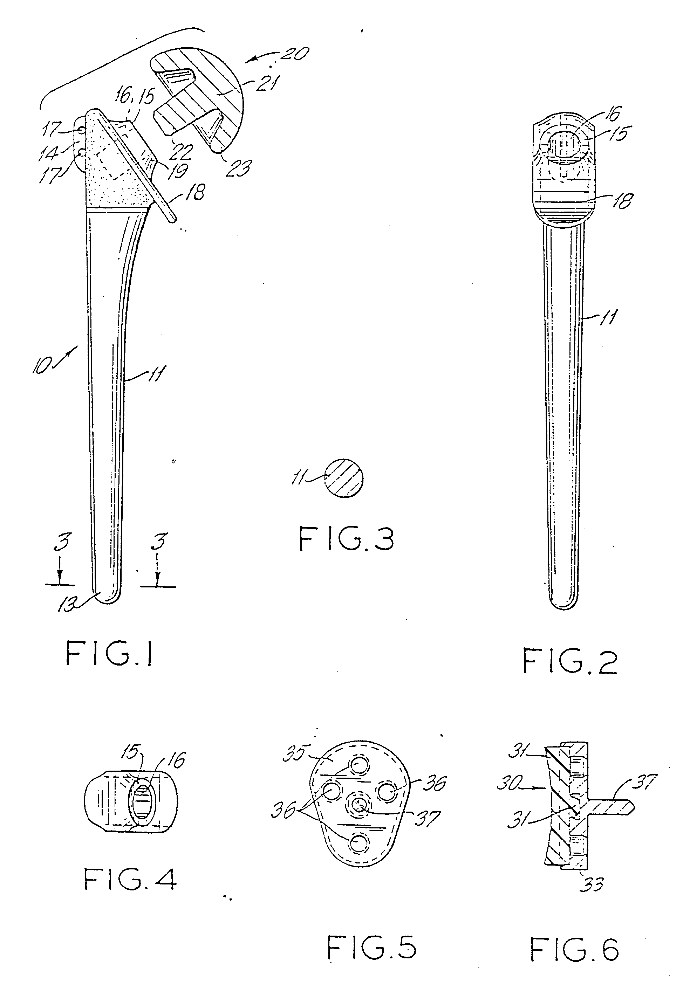

Referring now to Figs. 1-4, the humeral component according to the present invention includes stem 10 having an elongated portion 11 optionally including a rib on both sides thereof which aids in the fixation of the stem in the humerus and stability against rotation. The stem length is preferably in two sizes of 70 mm and 115 mm, and the stem width is in four sizes of 6 mm, 7 mm, 9 mm and 11 mm.

At the free end of the stem, there is a rounded portion 13 and at the upper end of the stem is a projecting surface 15 projecting from plane 18 to plane 19 and having bore 16 extending inwardly from the top plane thereof. The plane 15 is preferably at 45° or 60° to the axis of the stem.

The stem also includes a flange portion 14 including fixation holes 17 which aid in the fixation of the implant after embedded in the humerus.

The entire stem portion is preferably coated with a porous material for aiding in the fixation.

The head 20 shown in Fig. 1 includes a spherical sectional body 21 having a bottom flange 23 which abuts plane 18 and a stem 22 for inserting into bore 16 in a tightly fitting manner. Both the bore 16 and neck 22 are tapered in a reverse Morse taper and provide a friction fit when placed in a connected condition.

The head 21 has a diameter in one of four sizes including 28 mm, 40 mm, 44 mm and 48 mm and the height of the head is preferably 15 mm to 27 mm.

Figs. 5 and 6 show the glenoid component 30 in more detail including semi-spherical plastic seating surface 31 for engaging head 20 and stem portion 22 a "christmas tree" which is cut and is inserted into base 33 which has a stem 37 for inserting into bore and screw holes 36 for fixation.

The base of the glenoid component is preferably metal on plastic with a porous coating on the stem 22.

## Claims

1. "A shoulder prosthesis, comprising a glenoid component (30) and a humeral component (10, 20), the humeral component having an elongated stem (10), a semispherical head (20) and means removably connecting the head (20) to the proximal end of the stem (10), the distal portion of the stem (10) being for insertion into a humerus, characterized in

   that the proximal end portion of the elongated stem has a plane (18) having a raised collar with a planar upper surface (15) formed thereon, said planar upper surface (15) having a tapered aperture (16) formed therein and extending downwardly through said collar and into said proximal end portion of the stem; and that the semispherical head (20) has a concave recessed inner surface, said inner surface having a tapered pin (22) extending from a bottom portion of said recess and outwardly thereof and dimensioned so as to form a clearance therearound with said inner surface, wherein the recess is configured to receive and surround the raised collar when said tapered pin (22) is tightly received in said aperture (16) thereby securing said joint head (20) to the stem (10)."

2. The shoulder prosthesis according to claim 1 wherein the stem (10) tapers in cross section (11) from the proximal end toward the distal end.

3. The shoulder prosthesis according to claim 1, wherein the distal end portion of the stem is rounded (13).

4. The shoulder prosthesis according to claim 1, wherein the stem (10) is covered with a porous coating.

5. The shoulder prosthesis according to claim 1, wherein the stem (10) has a flange (14) in the vicinity of said proximal end portion thereof with fixation holes (17) therein.

6. The shoulder prosthesis according to claim 1, wherein the stem (10) has a length in the range of 70 to 115 mm and a width of from 6 to 11 mm.

7. The shoulder prosthesis according to claim 1, wherein the head (20) has a diameter from 20 to 48 mm and a thickness of 15 to 27 mm.

8. The shoulder prosthesis according to claim 1, wherein the stem (10) defines a longitudinal axis and the planar surface (15) extends from 45° to 60° to the axes of the stem.

## Patentansprüche

1. Schulterprothese mit einer Glenoidkomponente (30) und einer Eumeralkomponente (10, 20), wobei die Humeralkomponente einen länglichen Schaft (10), einen halbkugelförmigen Kopf (20) und den Kopf (20) mit dem proximalen Ende des Schaftes (10) lösbar verbindende Einrichtungen aufweist und der distale Abschnitt des Schaftes (10) zum Einsetzen in einen Humerus dient,
dadurch gekennzeichnet, daß
der proximale Endabschnitt des länglichen Schaftes eine Ebene (18) mit einer erhabenen Manschette mit einer darauf ausgebildeten planaren oberen Fläche (15) besitzt, in der eine konische Öffnung (16) ausgebildet ist, die sich nach unten durch die Manschette und in den proximalen Endabschnitt des Schaftes erstreckt; und der halbkugelförmige Kopf (20) eine konkave vertiefte Innenfläche aufweist, die einen konischen Stift (22) besitzt, der sich von einem unteren Abschnitt der Vertiefung nach außen erstreckt und so dimensioniert ist, daß er um den Stift herum in bezug auf die Innenfläche Spiel aufweist, wobei die Vertiefung so ausgebildet ist, daß sie die erhabene Manschette aufnimmt und umgibt, wenn der konische Stift (22) eng anliegend in der Öffnung (16) aufgenommen ist, wodurch der Gelenkkopf (20) am Schaft (10) befestigt wird.

2. Schulterprothese nach Anspruch 1, bei der sich der Schaft (10) im Querschnitt (11) vom proximalen Ende zum distalen Ende verjüngt.

3. Schulterprothese nach Anspruch 1, bei der der distale Endabschnitt des Schaftes abgerundet (13) ist.

4. Schulterprothese nach Anspruch 1, bei der der Schaft (10) mit einem porösen Überzug versehen ist.

5. Schulterprothese nach Anspruch 1, bei der der Schaft (10) in der Nachbarschaft seines proximalen Endabschnittes einen Flansch (14) mit darin ausgebildeten Fixierlöchern (17) aufweist.

6. Schulterprothese nach Anspruch 1, bei der der Schaft (10) eine Länge in einem Bereich von 70 bis 115 mm und eine Breite von 6 bis 11 mm besitzt.

7. Schulterprothese nach Anspruch 1, bei der der Kopf (20) einen Durchmesser von 20 bis 48 mm und eine Dicke von 15 bis 27 mm aufweist.

8. Schulterprothese nach Anspruch 1, bei der der Schaft (10) eine Längsachse bildet und sich die Planare Fläche (15) unter einem Winkel von 45° bis 60° zur Achse des Schaftes erstreckt.

## Revendications

1. Une prothèse de l'épaule comprenant un composant glénoïde (30) et un composant huméral (10, 20), le composant huméral ayant une tige allongée (10), une tête hémisphérique (20) et des moyens reliant de manière démontable la tête (20) à l'extrémité proximale de la tige (10), la portion distale de la tige (10) étant pour une insertion dans un humérus,
caractérisée en ce que la portion d'extrémité proximale de la tige allongée a un plan (18) ayant une collerette dressée vers le haut avec une surface supérieure plane (15) formée dessus, ladite surface supérieure plane (15) ayant une ouverture conique (16) formée dedans et s'étendant vers le bas à travers ladite collerette et dans ladite portion d'extrémité proximale de la tige ; et que la tête hémisphérique (20) a une surface interne concave évidée, ladite surface interne ayant une broche conique (22) s'étendant depuis une portion de fond dudit évidement et vers l'extérieur par rapport à lui et étant dimensionnée de manière à former un dégagement tout autour avec ladite surface interne, dans lequel l'évidement est configuré pour recevoir et entourer la collerette dressée vers le haut lorsque ladite broche conique (22) est reçue étroitement dans ladite ouverture (16) en fixant ainsi ladite tête assemblée (20) à la tige (10).

2. La prothèse d'épaule selon la revendication 1, dans laquelle la tige (10) diminue en section transversale (11) de l'extrémité proximale vers l'extrémité distale.

3. La prothèse d'épaule selon la revendication 1, dans laquelle la portion d'extrémité distale de la tige est arrondie (13).

4. La prothèse d'épaule selon la revendication 1, dans laquelle la tige (10) est couverte par un revêtement poreux.

5. La prothèse d'épaule selon la revendication 1, dans laquelle la tige (10) a une bride (14) au voisinage de sa dite portion d'extrémité proximale avec des trous de fixation (17) dedans.

6. La prothèse d'épaule selon la revendication 1, dans laquelle la tige (10) a une longueur dans la plage de 70 à 115 mm et une largeur allant de 6 à 11 mm.

7. La prothèse d'épaule selon la revendication 1, dans laquelle la tête (20) a un diamètre de 20 à 48 mm et une épaisseur de 15 à 27 mm.

8. La prothèse d'épaule selon la revendication 1, dans laquelle la tige (10) définit un axe longitudinal et la surface plane (15) s'étend de 45° à 60° par rapport à l'axe de la tige.

FIG.1

FIG.2

FIG.3

FIG.4

FIG.5

FIG.6